# EUROPÄISCHE PATENTSCHRIFT

(11) **EP 0 390 066 B1**
(45) Veröffentlichungstag und Bekanntmachung des Hinweises auf die Patenterteilung: **09.10.1996**
(21) Anmeldenummer: 90105798.4
(22) Anmeldetag: 27.03.1990
(51) Int. Cl.: C07D 501/34, A61K 31/545

(54) **Cephalosporinderivate und Verfahren zu ihrer Herstellung**
Cephalosporinderivatives and process for their preparation
Dérivés de céphalosporines et procédé pour leur préparation

(30) Priorität: 29.03.1989 DE 3910093
(43) Veröffentlichungstag der Anmeldung: 03.10.1990
(73) Patentinhaber: HOECHST AKTIENGESELLSCHAFT, 65926 Frankfurt am Main (DE)
(72) Erfinder: Friedhelm, Adam, Dr., D-6238 Hofheim am Taunus (DE); Dürckheimer, Walter, Dr., D-6234 Hattersheim am Main (DE); Fischer, Gerd, Dr., D-6230 Frankfurt am Main 80 (DE); Mencke, Burkhard, Dr., D-5409 Holzappel (DE); Seibert, Gerhard, Prof.Dr., D-6100 Darmstadt (DE); Isert, Dieter, Dr., D-6236 Eschborn (DE); Klesel, Norbert, Dr., D-6103 Griesheim (DE)

(56) Entgegenhaltungen:
- EP-A- 0 034 536
- EP-A- 0 049 119
- FR-A- 2 385 722

## Beschreibung

Die Erfindung betrifft neue Cephalosporinderivate, die besonders für die orale Applikation geeignet sind, ein Verfahren zu ihrer Herstellung und pharmazeutische Zubereitungen, die solche Verbindungen enthalten.

Obwohl viele klinisch relevanten Cephalosporine mit breitem antibakteriellen Spektrum entwickelt worden sind, eignen sich die meisten von ihnen nur für eine parenterale Verabreichung, da sie nach oraler Gabe, wenn überhaupt, nur sehr unzureichend resorbiert werden. In vielen Fällen ist es jedoch wünschenswert, dem Patienten hochwirksame Antibiotika in oraler Form zu geben.

Die bislang bekannten Cephalosporin-Antibiotika erfüllen nicht alle Anforderungen, die an ein solches Medikament gestellt werden müssen, nämlich eine hohe antibakterielle Aktivität gegen Gram-positive (speziell Staphylokokken) und Gram-negative Erreger und gleichzeitig eine gute Resorption im Magen-Darm-Trakt.

In einigen Fällen ist es gelungen, die Resorption eines Cephalosporins im Gastrointestinaltrakt durch Veresterung der 4-Carboxylgruppe zu erhöhen. Da die Cephalosporinester in der Regel per se keine antiobiotische Wirksamkeit aufweisen, muß die Esterkomponente so gewählt werden, daß der Ester nach der Resorption durch körpereigene Enzyme, wie Esterasen, wieder rasch und vollständig zum Cephalosporin mit freier Carboxylgruppe zurückgespalten wird.

Das Ausmaß der enteralen Resorption von Cephalosporinen ist maßgeblich abhängig von der chemischen Struktur des Cephalosporins und der jeweiligen Esterkomponente. Schon kleine strukturelle Variationen am Cephalosporin-Grundgerüst oder in der Esterkomponente können die Resorption beeinflussen. Das Auffinden geeigneter Komponenten ist rein empirisch.

So führt beispielsweise die Einführung eines sauren Substituenten in die 7β-Seitenkette von Aminothiazolyl-Cephalosporinen, wie z.B. im Cefixime, zu einer enteral resorbierbaren Verbindung, während Verbindungen mit neutralen Seitenketten, wie z.B. im Cefuroxim, nur in Form von Prodrug-Estern enteral resorbiert werden. Die Dosis-Wirkungs-Proportionalität ist dabei oft nicht linear und die erzielten therapeutischen Serumspiegel sind nicht befriedigend. Weitere Ester aus der Reihe der Aminothiazolyl-Cephalosporine werden beispielsweise in dem EP 34 536 erwähnt.

Wir fanden nun durch systematisch durchgeführte in vivo-Untersuchungen in verschiedenen Tierspecies eine enge Gruppe von oral applizierbaren Ceph-3-em-4-carbonsäureestern, die eine ausreichende chemische Stabilität besitzen und durch ausgewogene Lipid- und Wasserlöslichkeit rasch und in therapeutisch beachtlichem Maße im Magen-Darm-Trakt resorbiert werden.

Gegenstand der Erfindung sind demnach Cephemcarbonsäureester der allgemeinen Formel I 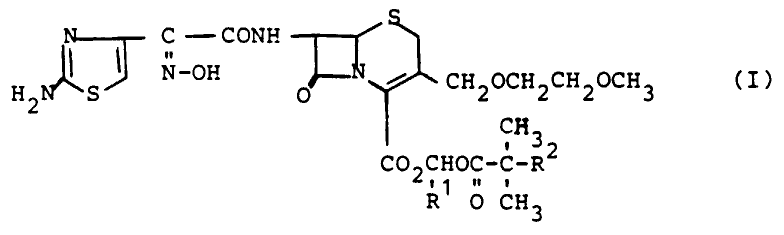 worin bedeuten
R¹ = Wasserstoff oder Methyl,
R² = Methyl oder Ethyl
und worin die HO-Gruppe in syn-Position steht und deren physiologisch verträgliche Säureadditionssalze.

Als physiologisch verträgliche Säureadditionssalze kommen die für Cephalosporin-Antibiotika bekannten Salze in Betracht, wie z.B. das Hydrochlorid, Sulfat, Maleinat, Citrat, Acetat oder Formiat. Ihre Herstellung erfolgt in an sich bekannter Weise durch Zusammengeben der Komponenten in einem wäßrigen oder organischen Lösungsmittel oder einer geeigneten Lösungsmittelmischung.

Die Verbindungen der allgemeinen Formel I besitzen für R¹ = CH₃ im Esterteil ein chirales Zentrum. Bei Verwendung racemischer Verbindungen der allgemeinen Formel III liegen die Cephemcarbonsäureester der allgemeinen Formel I als ein Gemisch von zwei Diastereomeren vor, die nach bekannten Methoden in die beiden Einzelkomponenten trennbar sind.

Gegenstand der Erfindung ist weiterhin ein Verfahren zur Herstellung von Cephemcarbonsäureestern der allgemeinen Formel I 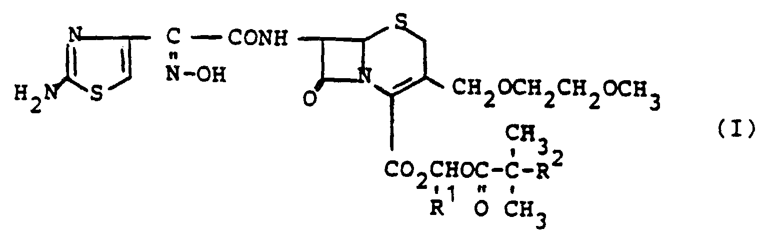 worin bedeuten
R¹ = Wasserstoff oder Methyl,
R² = Methyl oder Ethyl
und worin die HO-Gruppe in syn-Position steht und von deren physiologisch verträglichen Säureadditionssalzen, das dadurch gekennzeichnet ist, daß man
a) eine Verbindung der Formel II 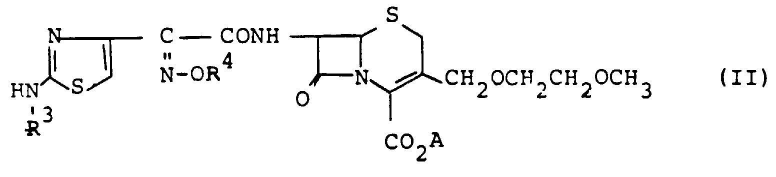 in der R³ für eine Aminoschutzgruppe, R⁴ eine leicht abspaltbare Gruppe und A ein Kation steht,
   mit einer Verbindung der allgemeinen Formel III 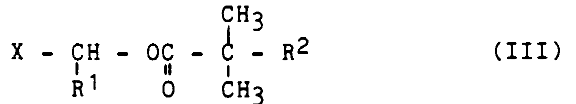 worin R¹ und R² die obige Bedeutung besitzen und X für eine Abgangsgruppe steht, zu dem Ester der allgemeinen Formel IV umsetzt 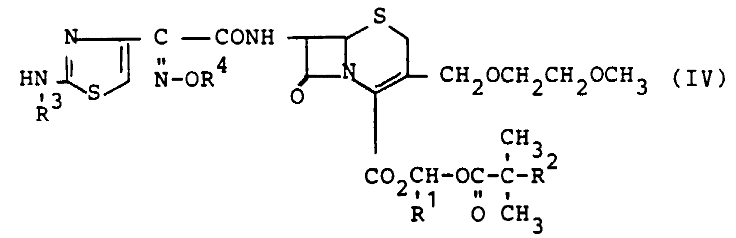 und die Gruppen R³ und R⁴ in an sich bekannter Weise entfernt
   oder
b) eine Verbindung der allgemeinen Formel V 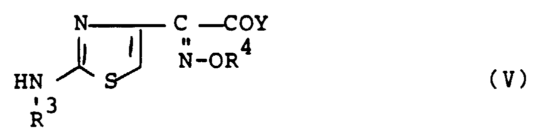 in der R³ und R⁴ die vorstehende Bedeutung besitzen und Y für eine aktivierende Gruppe steht, mit einer Verbindung der allgemeinen Formel VI 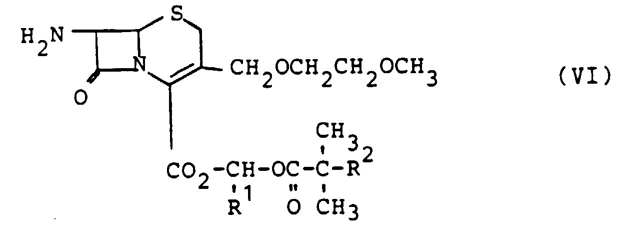 in der R¹ und R² die vorstehende Bedeutung besitzen oder mit einem Salz dieser Verbindung, zu einer Verbindung der allgemeinen Formel IV umsetzt und die Gruppen R³ und R⁴ in an sich bekannter Weise abspaltet,
   oder
c) eine Verbindung der allgemeinen Formel VII 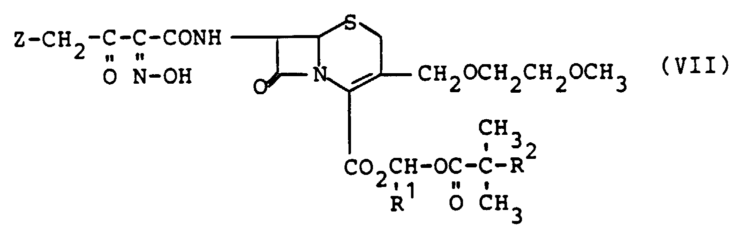 in der Z für Halogen steht und R¹ und R² die vorstehende Bedeutung besitzen, mit Thioharnstoff zu Verbindungen der allgemeinen Formel I umsetzt,
   und - falls erwünscht - die erhaltenen Verbindungen in ein physiologisch verträgliches Säureadditionssalz überführt.

In den Formeln II, IV und V steht R³ für eine aus der Peptid- und Cephalosporinchemie bekannte Aminoschutzgruppe, vorzugsweise für Formyl, Chloracetyl , Bromacetyl, Trichloracetyl, Benzyloxycarbonyl, tert.-Butoxycarbonyl oder Trityl und R⁴ für eine ebenfalls aus der Peptid- und Cephalosporinchemie bekannte leicht abspaltbare Gruppe, vorzugsweise für Benzhydryl, Trityl, Tetrahydropyranyl oder 1-Methoxy-1-methyl-ethyl. Besonders bevorzugt für R³ ist Trityl, für R⁴ Trityl und 1-Methoxy-1-methyl-ethyl.

In Formel III hat X die Bedeutung einer für Veresterungen allgemein bekannten Abgangsgruppe, wie beispielsweise Chlor, Brom, Jod, Phenylsulfonyloxy, p-Toluol-sulfonyloxy oder Methylsulfonyloxy, vorzugsweise Chlor, Brom oder Jod, insbesondere Jod.

Als Basen, die dem Kation A in der allgemeinen Formel II zugrundeliegen, seien z.B. genannt Natriumhydrogencarbonat, Kaliumhydrogencarbonat, Natriumcarbonat, Kaliumcarbonat, gegebenenfalls substituierte, alkylierte Aminbasen, wie z.B. Trimethylamin, Triethylamin, Diisopropylamin, Ethyldiisopropylamin, N,N-Dimethylanilin, N,N-Dimethylbenzylamin, 1,5-Diazabicyclo[4,3,0]non-5-en (DBN), 1,8-Diazabicyclo[5,4,0]undec-7-en (DBU), Pyridin, Picolin oder 2,6-Dimethylpyridin. Bevorzugte Basen sind Natrium- oder Kaliumhydrogencarbonat, Natrium- oder Kaliumcarbonat, Triethylamin, N,N-Dimethylanilin, DBN oder DBU.

Durch Umsetzung der freien Carbonsäuren mit diesen Basen erhält man die Salze der allgemeinen Formel II, in der A für ein Kation, wie beispielsweise Natrium oder Kalium, aber auch für Magnesium oder Calcium oder für ein gegebenenfalls substituiertes alkyliertes Ammoniumion, wie beispielsweise Ammonium, Trimethylammonium, Triethylammonium, Tetrabutylammonium, Diisopropylammonium, Ethyldiisopropylammonium, Diazabicyclo [0,3,4] nonenium oder Diazabicyclo [0,4,5] undecenium steht. Bevorzugte Bedeutungen von A sind Natrium, Kalium, Triethylammonium, N,N-Dimethylanilinium, sowie das DBN- oder DBU-ion.

In Verbindungen der Formel VII steht Z für ein Halogenatom, vorzugsweise für Brom oder Chlor.

Die Umsetzung der Verbindungen der Formel II mit den Verbindungen der Formel III kann in einem organischen Lösungsmittel bei etwa -20 bis etwa +50°C, bevorzugt bei etwa 0°C bis Raumtemperatur durchgeführt werden. Als Lösungsmittel können beispielsweise dienen Ketone, wie beispielsweise Aceton oder Methylethylketon, N,N-Dimethylformamid (DMF), N,N-Dimethylacetamid (DMA), N-Methylpyrrolidon oder Dimethylsulfoxid (DMSO). Bevorzugt sind DMF, DMA, N-Methylpyrrolidon und DMSO. Besonders bevorzugt ist DMF.

Die Abspaltung der Gruppen R³ und R⁴ aus den erhaltenen Verbindungen der Formel IV erfolgt in aus der Peptid- und Cephalosporinchemie an sich bekannter Weise, z.B. mit Trifluoressigsäure, verdünnter Salzsäure, vorzugsweise mit Ameisensäure unter Zugabe von etwas Wasser.

Setzt man eine Verbindung der Formel V mit einer Verbindung der Formel VI um, so stellt Y eine die Carboxylgruppe aktivierende Gruppe dar, wie sie aus der Peptid- und Cephalosporinchemie für entsprechende Reaktionen bekannt ist, beispielsweise ein Halogenid, vorzugsweise Chlorid, eine aktivierende Estergruppe, beispielsweise mit 1-Hydroxybenzotriazol oder ein gemischtes Anhydrid, beispielsweise mit Benzolsulfonsäure oder Toluolsulfonsäure. Die Aktivierung der Carboxylgruppe ist auch in literaturbekannter Weise über die Zugabe eines Kondensationsmittels, wie z. B eines Carbodiimids, möglich.

Die Verbindung der allgemeinen Formel VI läßt sich als solche oder in Form eines Salzes, beispielsweise des Tosylats, Hydrochlorids oder Hydrojodids einsetzen, wobei die Verwendung kristalliner Salze im Hinblick auf die Reinheit der Produkte vorteilhaft sein kann.

Die Umsetzung von Verbindungen der Formel V mit solchen der Formel VI kann in einem organischen Lösungsmittel, wie beispielsweise Methylenchlorid, Chloroform, Aceton, Methylethylketon, Dimethylformamid, Dimethylacetamid oder Wasser, oder auch in Mischungen dieser Lösungsmittel erfolgen.

Die Acylierungsreaktion kann zweckmäßigerweise bei Temperaturen von etwa -50°C bis etwa +50°C, vorzugsweise -40°C bis +30°C, gewünschtenfalls in Anwesenheit einer Base, wie beispielsweise Triethylamin oder Pyridin, durchgeführt werden. Der Basenzusatz dient dazu, die bei der Kondensation freiwerdende acide Komponente zu binden.

Der Ringschluß von Verbindungen der allgemeinen Formel VII mit Thioharnstoff kann nach an sich bekannten Verfahren, wie sie beispielsweise in der EP-PS 134 420 beschrieben sind, durchgeführt werden. Es gelingt z.B. glatt bei Temperaturen von etwa 0 bis 30°C, vorzugsweise etwa 5°C in organischen Lösungsmitteln, vorzugsweise aprotisch polaren Solventien, wie z.B. Dimethylformamid, Dimethylacetamid, Acetonitril oder Aceton.

Die Ausgangsverbindungen der Formel III können in an sich bekannter Weise hergestellt werden, indem man Verbindungen der allgemeinen Formel 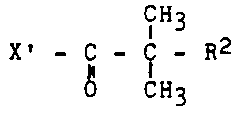 in der R² die oben angegebene Bedeutung hat und X′ für eine Abgangsgruppe steht, mit Aldehyden der Formel

R¹-CHO

in der R¹ die oben angegebene Bedeutung hat, umsetzt. Die bevorzugte Bedeutung von X′ ist Brom oder Chlor. Die Reaktion wird zweckmäßigerweise in einem organischen Lösungsmittel, wie einem halogenierten Kohlenwasserstoff, beispielsweise Methylenchlorid oder Chloroform, in Anwesenheit eines Katalysators, wie beispielsweise Zinkchlorid oder Aluminiumchlorid, bei einer Temperatur von zweckmäßigerweise -10°C bis +10°C durchgeführt.

Die Ausgangsverbindungen der Formel III, in der X für Chlor steht, können alternativ hergestellt werden, indem man eine Carbonsäure der Formel 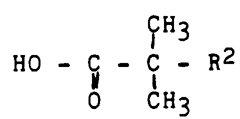 in der R² die oben angegebene Bedeutung hat mit einer Verbindung der Formel 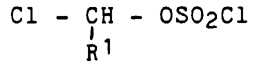 in der R¹ die oben angegebene Bedeutung hat und deren Herstellung in Synthetic Communications 14, S. 857 beschrieben wird, in Gegenwart einer Base wie beispielsweise Natriumcarbonat, Kaliumcarbonat, Natriumhydrogencarbonat oder Kaliumhydrogencarbonat, bevorzugt Natriumhydrogencarbonat, umsetzt. Die Reaktion wird bevorzugt bei 0°C bis Raumtemperatur in einem Zweiphasengemisch, vorzugsweise aus Wasser und einem chlorierten Kohlenwasserstoff, wie z.B. Methylenchlorid oder Chloroform, in Anwesenheit eines Phasentransferkatalysators, wie beispielsweise Tetrabutylammoniumhydrogensulfat, durchgeführt.

Die Ausgangsverbindungen der Formel III können auch durch Halogenaustausch hergestellt werden. So erhält man beispielsweise eine Verbindung der Formel III, in der X für Jod steht, durch Umsetzen der entsprechenden Verbindung III, in der X für Chlor oder Brom steht, mit einem Jodidsalz, wie beispielsweise Natriumjodid.

Die Herstellung von Ausgangsverbindungen der allgemeinen Formel V mit der aktivierten Carboxylgruppe erfolgt in literaturbekannter Weise, die zu den Verbindungen der Formel VI führende Veresterung auf dieselbe Weise, wie sie für die Herstellung der Ester der allgemeinen Formel IV beschrieben wurde.

Die Verbindungen der allgemeinen Formel VII lassen sich nach an sich bekannten Verfahren herstellen. So kann man beispielsweise (vgl. EP-PS 134 420) Diketen mit Brom, und das erhaltene Zwischenprodukt dann mit einer Verbindung der allgemeinen Formel VI umsetzen, wobei man ein Vorprodukt der Formel 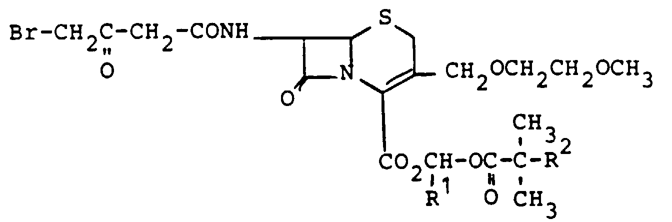 erhält, das anschließend durch Nitrosierung (vgl. ebenfalls EP-PS 134 420) in eine Verbindung der allgemeinen Formel VII überführt wird.

Die Ceph-3-em-4-carbonsäureester der allgemeinen Formel I besitzen eine Reihe physikochemischer und biologischer Eigenschaften, die sie zu wertvollen Cephalosporin-Antibiotika zur oralen Verabreichung machen. Sie sind stabile, farblose und in gängigen organischen Lösungsmitteln gut lösliche Verbindungen, die im Darm resorbiert, im Serum rasch zum antibiotisch aktiven Cephalosporinderivat der Formel 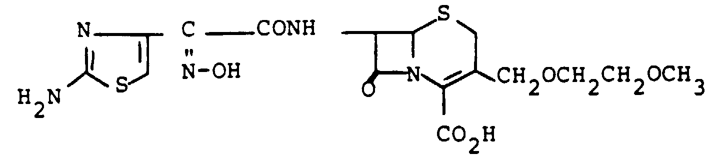 gespalten werden und sich daher hervorragend zur Behandlung von bakteriellen Infektionskrankheiten, wie z.B. der Infektion der Atemwege oder des Urogenitaltraktes eignen.

Die erfindungsgemäßen Verbindungen werden oral verabreicht in Form von üblichen pharmazeutischen Zubereitungen, wie z.B. Kapseln, Tabletten, Pulvern, Sirupen oder Suspensionen. Die Dosis hängt ab vom Alter, den Symptomen und dem Körpergewicht des Patienten sowie von der Dauer der Behandlung. Sie liegt jedoch in der Regel zwischen etwa 0,2 g und etwa 5 g täglich, vorzugsweise zwischen etwa 0,5 und etwa 3 g täglich. Die Verbindungen werden vorzugsweise in aufgeteilten Dosen verabreicht, beispielsweise 2 bis 4mal täglich, wobei die Einzeldosis beispielsweise zwischen 50 und 500 mg Wirkstoff enthalten kann.

Die oralen Zubereitungen können die üblichen Trägerstoffe und/oder Verdünnungsmittel enthalten. So kommen beispielsweise für Kapseln oder Tabletten in Betracht Bindemittel, wie z.B. Gelatine, Sorbitol, Polyvinylpyrrolidon oder Carboxymethylcellulose, Verdünnungsmittel, wie z.B. Lactose, Zucker, Stärke, Calciumphosphate oder Polyethylenglykol, Gleitstoffe, wie z.B. Talkum oder Magnesiumstearat, für flüssige Zubereitungen z.B. wäßrige oder ölige Suspensionen, Sirupe oder ähnliche bekannte Zubereitungsformen.

Die folgenden Beispiele dienen zur weiteren Erläuterung der Erfindung, schränken sie jedoch nicht darauf ein.

### Ausführungsbeispiele

### 7-[2-(2-Aminothiazol-4-yl)-2-(Z)-hydroxyimino-acetamido]-3-(2-methoxyethoxy-methyl)-3-cephem-4-carbonsäure-α-(2,2-dimethyl-propanoyl-oxy)-ethylester

### Verfahrensvariante a)

### Stufe 1

### 7-[2-(2-Tritylaminothiazol-4-yl)-2-(Z)-trityl-oximino-acetamido]-3-(2-methoxyethoxy-methyl)-3-cephem-4-carbonsäure-α-(2,2-dimethyl-propanoyl-oxy)-ethylester

Eine Lösung von 14 g (14,8 mmol) 7-[2-(2-Tritylaminothiazol-4-yl)-2-(Z)-trityl-oximinoacetamido]-3-(2-methoxyethoxy-methyl)-3-cephem-4-carbonsäure in 300 ml wasserfreiem Dimethylformamid wurden mit 1,07 g (7,7 mmol) Kaliumcarbonat versetzt und bis zur Auflösung des Salzes bei Raumtemperatur gerührt. Danach kühlte man im Eisbad und gab 4,4 g α-2,2-Dimethyl-propionsäure-jodethylester zu. Man rührte noch 2 Stunden bei 0°C nach, zog das Lösungsmittel im Vakuum ab und verteilte zwischen Essigester und Wasser. Die organische Phase wurde über Magnesiumsulfat getrocknet, die Lösung eingeengt und der feste Rückstand chromatographiert (SiO₂; Toluol/EE = 10 + 1). Man erhielt 5 g reine Titelverbindung als Gemisch der beiden Diastereomeren.

¹H-NMR (270 MHz, d⁶-DMSO): δ = 1.12 und 1.13 (9H, s, -C(CH₃)₃, 1.46 (3H, dd, CH(CH₃), 3.23 (3H, s, OCH₃), 3.4 - 3.61 (6H, m, S-CH₂ und O-CH₂CH₂-OCH₃), 4.2 (2H, d,__3-CH₂), 5.18 (1H, 2dd, 6-H), 5.83 (1H, dt, 7-H), 6.65 (1H, d, Thiazol-H), 6.9 (1H, dq, J = 6Hz, CH(CH₃)), 7.1 (2H, breites s, NH₂), 9.45 (1H, dd, J = 6Hz, NH), 11.27 (1H, s, Oxim-H).

Die Weiterverarbeitung erfolgte nach Stufe 2 (siehe unten).

### Verfahrensvariante b)

### Vorstufe

### 2-(2-Tritylaminothiazol-4-yl)-2-(Z)-(1-methyl-1-methoxy)-ethoxy-imino-essigsäure-p-toluolsulfonsäureanhydrid

Zu einer Suspension von 6 g (10 mmol) 2-(2-Tritylaminothiazol-4-yl)-2-(Z)-(1-methyl-1-methoxy)-ethoxyimino-essigsäure-triethyl-ammoniumsalz in 30 ml Aceton wurden 2,1 g (11 mmol) p-Toluolsulfonsäurechlorid gegeben und 1,5 Stunden bei Raumtemperatur gerührt. Anschließend versetzte man mit 40 ml Diethylether, kühlte auf -10°C und saugte danach ab. Das Produkt wurde noch dreimal mit je 20 ml Ether nachgewaschen und getrocknet. Man erhielt 10 g Produkt, das aus einem Gemisch der Titelverbindung und Triethylaminohydrochlorid bestand und ohne weitere Reinigung weiterverarbeitet wurde.

### Stufe 1

### 7-[2-(2-Tritylaminothiazol-4-yl)-2-(Z)-(1-methyl-1-methoxy)- ethoxyiminoacetamido ]-3-(2-methoxyethoxy-methyl)-3-cephem-4-carbonsäure-α-(2,2-dimethylpropanoyloxy)-ethylester

2 g (7 mmol) 7-Amino-3-(2-methoxyethoxy-methyl)-ceph-3-em-4-carbonsäure wurden in 20 ml Methylenchlorid suspendiert und 0,7 ml (4,9 mmol) 1,8-Diazabicyclo-(5,4,0)-undec-7-en (DBU) bei 0°C versetzt. Zu dieser Lösung gab man 1,8 g (7 mmol) α-2,2-Dimethyl-propionsäure-jodethylester und rührte 30 Minuten bei 0°C nach. Danach gab man 3,3 g (3,5 mmol) des in der Vorstufe erhaltenen gemischten Anhydrids zu und rührte die Lösung 30 Minuten bei Raumtemperatur nach.

Nach beendeter Reaktion wurde das Lösungsmittel im Vakuum abgezogen und der Ruckstand zwischen Essigester und Wasser verteilt. Die organische Phase wurde 1 × mit 5 %iger Natriumbicarbonat-Lösung gewaschen, über Magnesiumsulfat getrocknet und das Lösungsmittel im Vakuum abgezogen. Der Rückstand wurde chromatographiert (SiO₂; Toluol/Essigester = 3 : 1). Man erhielt 2,5 g (40 %) der gewünschten Verbindung, die ohne weitere Charakterisierung in der nächsten Stufe umgesetzt wurde.

### Stufe 2

### 7-[2-(2-Aminothiazol-4-yl)-2-(Z)-hydroxyimino-acetamido]-3-(2-methoxyethoxy-methyl)-3-cephem-4-carbonsäure-α-(2,2-dimethyl-propanoyloxy)-ethylester

2,5 g (2,8 mmol) der in Stufe 1 erhaltenen Verbindung wurden in 32 ml Ameisensäure gelöst und anschließend mit 8 ml Wasser versetzt. Nach 1 Stunde Rühren bei Raumtemperatur wurde vom entstehenden Triphenylcarbinol abgesaugt und das Filtrat im Vakuum eingeengt. Anschließend nahm man das Rohprodukt in Essigester auf, extrahierte einmal mit gesättigter Natriumhydrogencarbonatlösung, trocknete die organische Phase über Magnesiumsulfat und rotierte ein. Man erhielt 1 g (61 %) Produkt, das in 5 ml Essigester gelöst und in 50 ml Diisopropylether eingetropft wurde. Man erhielt danach 735 mg der gewünschten Titelverbindung als ein Gemisch der beiden Diastereomeren, das in allen Eigenschaften mit dem nach Verfahrensvariante a) erhaltenen Produkt identisch war.

In analoger Weise zu Ausführungsbeispiel 1, Verfahrensvariante a) oder b) wurden folgende Verbindungen erhalten:

### 7-[2-(2-Aminothiazol-4-yl)-2-(Z)-hydroxyimino-acetamido]-3-(2-methoxyethoxy-methyl)-3-cephem-4-carbonsäure-2,2-dimethylpropanoyl-oxymethylester

¹H-NMR (270 MHz, DMSO-d₆): δ (ppm) = 1.15 (9H, s, C(CH₃)₃), 3.23 (3H, s, -OCH₃), 3.32 - 3.65 (6H, m, SCH₂ und -OCH₂CH₂-OCH₃), 4.21 (2H, s, CH₂-OR), 5.2 (1H, d, J = 6Hz, 6-H), 5.77 - 5.9 (3H, m, 7-H und O-CH₂-OCO-), 6.67 (1H, s, Thiazol-H), 7.1 (2H, breites s, NH₂), 9.46 (1H, d, J = 8Hz, NH), 11.3 (1H, breites s, Oxim-H).

### 7-[2-(2-Aminothiazol-4-yl)-2-(Z)-hydroxyimino-acetamido]-3-(2-methoxyethoxy-methyl)-3-cephem-4-carbonsäure-2,2-dimethylbutanoyloxymethylester

¹H-NMR (270 MHz, DMSO-d₆): δ (ppm) = 0,78 (3H, t, CH₂CH₃), 1.14 (6H, s, (CH₃)₂), 1.55 (2H, q, CH₂CH₃), 3.2 (3H, s, OCH₃), 3.4 - 3.6 (6H, m, S-CH₂ und -OCH₂CH₂OCH₃), 4.21 (2H, d, 3-CH₂), 5.22 (1H, dd, J = 6Hz, 6-H), 5.8 (3H, m, 7-H und -OCH₂OCO-), 6.66 (1H, d, Thiazol-H), 7.1 (2H, breites s, NH₂), 9.45 (1H, d, J = 7.5 Hz, NH), 11.3 (1H, breites s, Oxim-H).

### 7-[2-Aminothiazol-4-yl)-2-(Z)-hydroxyimino-acetamido]-3-(2-methoxyethoxy-methyl)-3-cephem-4-carbonsäure-α-(2,2-dimethyl-butanoyl-oxy)-ethylester

¹H-NMR (270 MHz, DMSO-d₆): δ (ppm): 0.77 (3H, t, CH₂-CH₃), 1.12 (6H, s, CH₃), 1.50 (5H, dq, CH(CH₃) und CH₂-CH₃), 3.22 (3H, s, OCH₃), 3.4 - 3.6 (6H, m, S-CH₂ und -OCH₂CH₂OCH₃), 4.2 (2H, d, 3-CH₂), 5.18 (1H, dd, 6-H), 5.81 (1H, dt, 7-H), 6.65 (1H, d, Thiazol-H), 6.9 (1H, dq, J = 6Hz, CH(CH₃), 7.1 (2H, breites s, NH₂), 9.45 (1H, dd, J = 6Hz, NH), 11.3 (1H, s, Oxim-H).

### Verfahrenvaraiante c)

### Vorstufe 1

### 7-Amino-3-(2-methoxyethoxy-methyl)-3-cephem-4-carbonsäure-α-(2,2-dimethylpropanoyloxy)-ethylester

2.9 g (10 mmol) 7-Amino-3-(2-methoxyethoxy-methyl)-ceph-3-em-4-carbonsäure wurden in 35 ml Methylenchlorid suspendiert und bei 0°c mit 1.3 ml (8.5 mmol) 1,8-Diazabicyclo-(5.4.0)-undec-7-en (DBU) versetzt. Nach 15 Minuten Nachrühren wurden unter Eiskühlung 2.6 g (10 mmol) ; α-2,2-Dimethyl-propionsäure-jodethylester zugetropft und 1 h bei 0°C nachgerührt. Danach filtrierte man ausgefallenen Niederschlag ab und verwendete die Lösung ohne weitere Isolierung in der nächsten Stufe.

### Vorstufe 2

### 7-(Bromacetyl-acetamido)-3-(2-methoxyethoxy-methyl)-3-cephem-4-carbonsäure-α-(2,2-dimethylpropanoyloxy) -ethylester

Zur einer Lösung von 0.75 ml (10 mmol) Diketen in 50 ml Methylenchlorid wurden bei - 40°C 0.5 ml (10 mmol) Brom zugetropft und 20 Minuten nachgerührt. Anschließend tropfte man bei dieser Temperatur die Lösung von Vorstufe 1 zu und arbeitete nach 45 Minuten auf. Dazu engte man ein, löste den anfallenden dunklen Rückstand in 20 ml Essigester und filtrierte über wenig Kieselgel (Cyclohexan/Essigester = 1 + 1). Nach Abzug des Lösungsmittels erhielt man 5.2 g Produkt, das ohne weitere Reinigung in vorstufe 3 weiterverarbeitet wurde.

DC: Rf - 0.7 (SiO₂; Toluol/Essigester = 2 + 1)

### Vorstufe 3

### 7-(2-Bromacetyl-2-hydroximino-acetamido)-3-(2-methoxyethoxy-methyl)-3-cephem-4-carbonsäure-α-(2,2-dimethylpropanoyloxy)-ethylester

Eine Lösung von 5.2 g der in Vorstufe 2 erhaltenen Verbindung in 50 ml Methylenchlorid und 18 ml Eisessig wurde auf - 10^{o}C gekühlt und mit 840 mg (12.3 mmol) Natriumnitrit in 8 ml Wasser versetzt. Nach 30 Minuten bei Raumtemperatur wurden 750 mg (12.6 mmol) Harnstoff zugegeben und nach weiteren 30 Minuten 45 ml Wasser. Danach trennte man die Phasen, wusch die organische Phase dreimal mit Wasser und einmal mit gesättigter Natriumchloridlösung und trocknete über Natriumsulfat. Nach Abzug des Lösungsmittels wurden 2.7g (44 % bezogen auf Vorstufe 1) der gewünschten Titelverbindung erhalten.

DC: Rf = 0.6 (SiO₂; Toluol/Essigester = 2 + 1)

### 7-[2-(2-Aminothiazol-4-yl)-2-(Z)-hydroximino-acetamido]-3-(2-methoxyethoxy-methyl)-3-cephem-4-carbonsäure-α-(2,2-dimethylpropanoyloxy)-ethylester

Zu einer Lösung von 2.7 g (4.4 mmol) der in Vorstufe 3 erhaltenen Verbindung in 30 ml Dimethylacetamid wurden bei 15^{o}C 500 mg Thioharnstoff zugegeben. Nach 1.5 h Rühren bei Raumtemperatur versetzte man mit 100 ml einer 3%igen Natriumhydrogencarbonatlösung, saugte den sich bildenden Niederschlag ab und nahm diesen in 100 ml Essigester auf. Anschließend wurde die organische Lösung mit gesättigter Natriumchloridlösung gewaschen, getrocknet (MgSO₄) und das Lösungsmittel i. Vak. abgezogen. Den Ruckstand löste man in 10 ml Essigester auf und tropfte die Lösung in 75 ml Diisopropylether. Das Produkt wurde abgesaugt, getrocknet. Man erhielt so 1.75 g (67 %) der gewünschten Titelverbindung als im Gemisch der beiden Diastereomeren, das in seinen Eigenschaften mit dem nach Verfahrensvarianten a) und b) erhaltenen identisch war.

## Patentansprüche (Patentansprüche für folgende(n) Vertragsstaat(en): AT, BE, CH, DE, DK, FR, GB, IT, LI, LU, NL, SE)

1. Cephemcarbonsäureester der allgemeinen Formel I 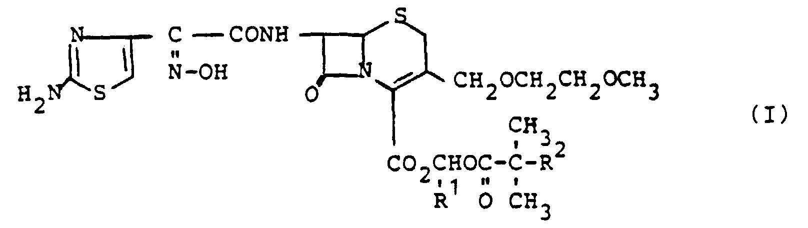 worin bedeuten
R¹ = Wasserstoff oder Methyl,
R² = Methyl oder Ethyl
und worin die HO-Gruppe in syn-Position steht und deren physiologisch verträgliche Säureadditionssalze.

2. 7-[2-(2-Aminothiazol-4-yl)-2-(Z)-hydroxyimino-acetamido]-3-(2-methoxyethoxy-methyl)-3-cephem-4-carbonsäure-2,2-dimethylpropanoyl-oxymethylester

3. 7-[2-(2-Aminothiazol-4-yl)-2-(Z)-hydroxyimino-acetamido]-3-(2-methoxyethoxy-methyl)-3-cephem-4-carbonsäure-2,2-dimethylbutanoyl-oxymethylester

4. 7-[2-(2-Aminothiazol-4-yl)-2-(Z)-hydroxyimino-acetamido]-3-(2-methoxyethoxy-methyl)-3-cephem-4-carbonsäure-α-(2,2-dimethylpropanoyl-oxy)-ethylester

5. 7-[2-(2-Aminothiazol-4-yl)-2-(Z)-hydroxyimino-acetamido]-3-(2-methoxyethoxy-methyl)-3-cephem-4-carbonsäure-α-(2,2-dimethylbutanoyl-oxy)-ethylester

6. Verfahren zur Herstellung von Cephemcarbonsäureestern der allgemeinen Formel I 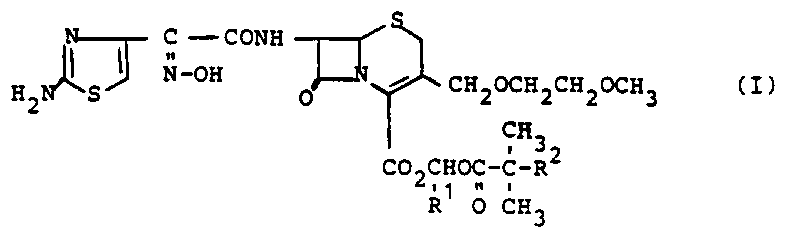 und deren physiologisch verträglichen Säureadditionssalzen worin bedeuten
R¹ = Wasserstoff oder Methyl,
R² = Methyl oder Ethyl
und worin die HO-Gruppe in syn-Position steht, dadurch gekennzeichnet, daß man
a) eine Verbindung der Formel II 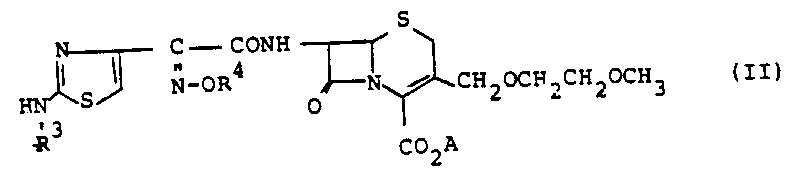 in der R³ für eine Aminoschutzgruppe, R⁴ eine leicht abspaltbare Gruppe und A ein Kation steht,
mit einer Verbindung der allgemeinen Formel III 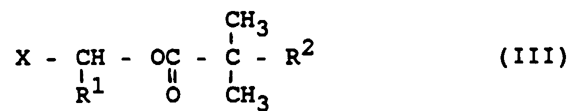 worin R¹ und R² die obige Bedeutung besitzen und X für eine Abgangsgruppe steht, zu dem Ester der allgemeinen Formel IV umsetzt 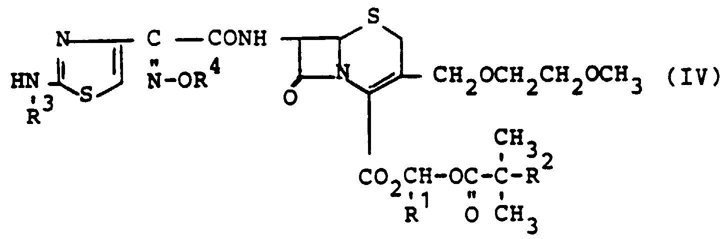 und die Gruppen R³ und R⁴ in an sich bekannter Weise entfernt
oder
b) eine Verbindung der allgemeinen Formel V 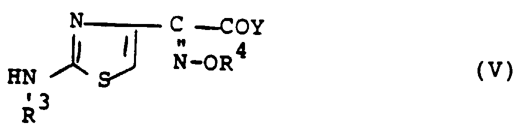 in der R³ und R⁴ die vorstehende Bedeutung besitzen und Y für eine aktivierende Gruppe steht, mit einer Verbindung der allgemeinen Formel VI 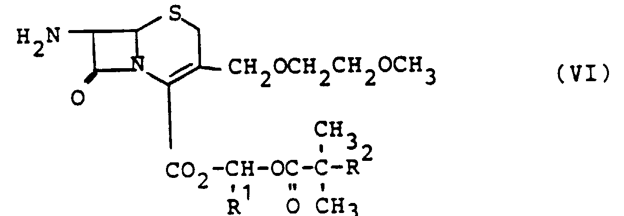 in der R¹ und R² die vorstehende Bedeutung besitzen oder mit einem Salz dieser Verbindung, zu einer Verbindung der allgemeinen Formel IV umsetzt und die Gruppen R³ und R⁴ in an sich bekannter Weise abspaltet, oder
c) eine Verbindung der allgemeinen Formel VII 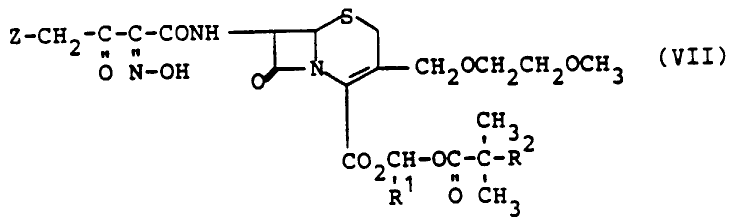 in der Z für Halogen steht und R¹ und R² die vorstehende Bedeutung besitzen, mit Thioharnstoff zu Verbindungen der allgemeinen Formel I umsetzt,
und - falls erwünscht - die erhaltenen Verbindungen in ein physiologisch verträgliches Säureadditionssalz überführt.

7. Gegen bakterielle Infektionen wirksame pharmazeutische Zubereitungen gekennzeichnet durch einen Gehalt an einem Cephemcarbonsäureester der allgemeinen Formel I.

8. Verfahren zur Herstellung von gegen bakterielle Infektionen wirksamen pharmazeutischen Zubereitungen, dadurch gekennzeichnet, daß ein Cephemcarbonsäureester der allgemeinen Formel I mit pharmazeutisch üblichen Trägerstoffen oder Verdünnungsmitteln in eine pharmazeutisch geeignete Verabreichungsform gebracht wird.

9. Verwendung von Cephemcarbonsäureestern der allgemeinen Formel I zur Herstellung eines Arzneimittels zur Bekämpfung bakterieller Infektionen.

## Patentansprüche (Patentansprüche für folgende(n) Vertragsstaat(en): ES, GR)

1. Verfahren zur Herstellung von Cephemcarbonsäureestern der allgemeinen Formel I 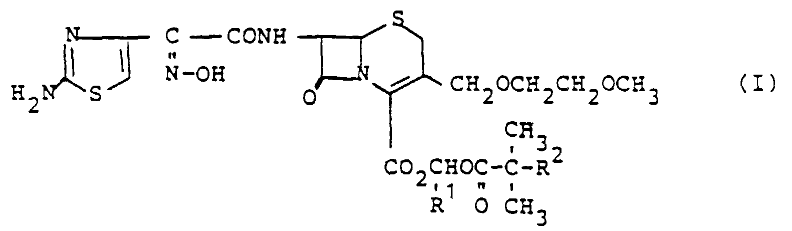 und deren physiologisch verträglichen Säureadditionssalzen worin bedeuten
R¹ = Wasserstoff oder Methyl,
R² = Methyl oder Ethyl
und worin die HO-Gruppe in syn-Position steht, dadurch gekennzeichnet, daß man
a) eine Verbindung der Formel II 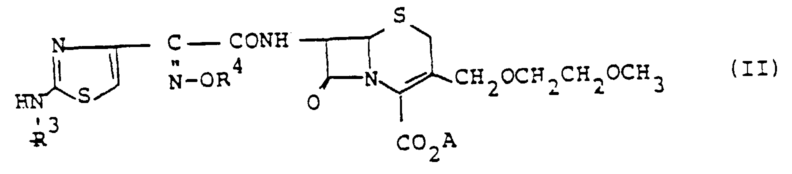 in der R³ für eine Aminoschutzgruppe, R⁴ eine leicht abspaltbare Gruppe und A ein Kation steht,
mit einer Verbindung der allgemeinen Formel III 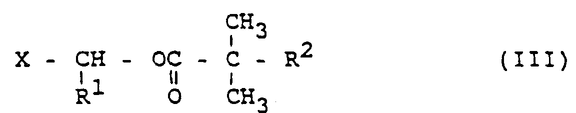 worin R¹ und R² die obige Bedeutung besitzen und X für eine Abgangsgruppe steht, zu dem Ester der allgemeinen Formel IV umsetzt 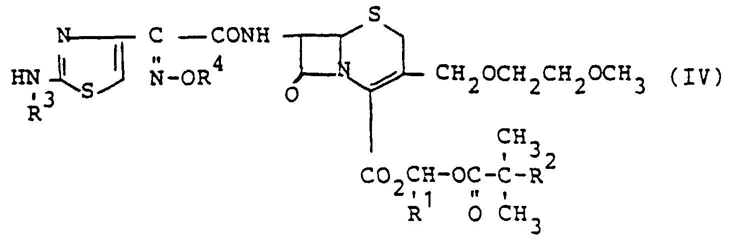 und die Gruppen R³ und R⁴ in an sich bekannter Weise entfernt
oder
b) eine Verbindung der allgemeinen Formel V 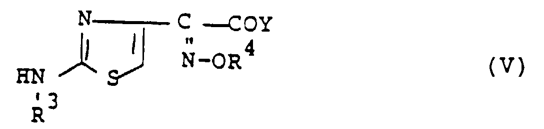 in der R³ und R⁴ die vorstehende Bedeutung besitzen und Y für eine aktivierende Gruppe steht, mit einer Verbindung der allgemeinen Formel VI 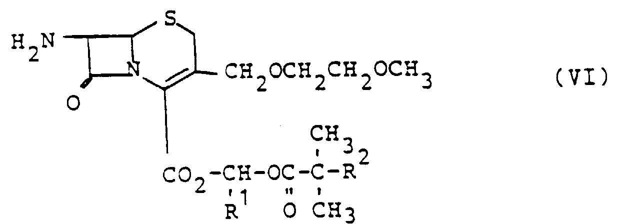 in der R¹ und R² die vorstehende Bedeutung besitzen oder mit einem Salz dieser Verbindung, zu einer Verbindung der allgemeinen Formel IV umsetzt und die Gruppen R³ und R⁴ in an sich bekannter Weise abspaltet, oder
c) eine Verbindung der allgemeinen Formel VII 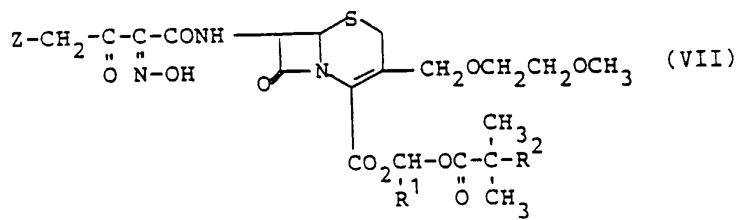 in der Z für Halogen steht und R¹ und R² die vorstehende Bedeutung besitzen, mit Thioharnstoff zu Verbindungen der allgemeinen Formel I umsetzt,
und - falls erwünscht - die erhaltenen Verbindungen in ein physiologisch verträgliches Säureadditionssalz überführt.

2. Gegen bakterielle Infektionen wirksame pharmazeutische Zubereitungen gekennzeichnet durch einen Gehalt an einem Cephemcarbonsäureester der allgemeinen Formel I.

3. Verfahren zur Herstellung von gegen bakterielle Infektionen wirksamen pharmazeutischen Zubereitungen, dadurch gekennzeichnet, daß ein Cephemcarbonsäureester der allgemeinen Formel I mit pharmazeutisch üblichen Trägerstoffen oder Verdünnungsmitteln in eine pharmazeutisch geeignete Verabreichungsform gebracht wird.

4. Verwendung von Cephemcarbonsäureestern der allgemeinen Formel I zur Herstellung eines Arzneimittels zur Bekämpfung bakterieller Infektionen.

## Claims (Claims for the following Contracting State(s): AT, BE, CH, DE, DK, FR, GB, IT, LI, LU, NL, SE)

1. A cephemcarboxylic acid ester of the formula I 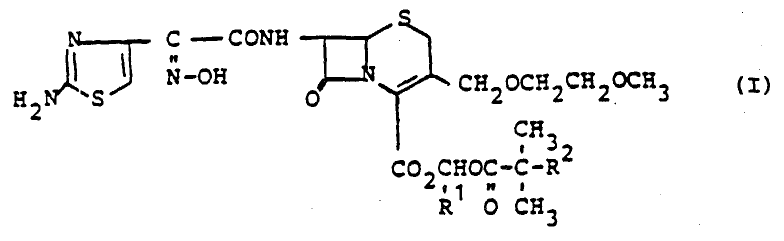 in which
R¹ = hydrogen or methyl,
R² = methyl or ethyl
and in which the HO group is in the syn-position, or a physiologically tolerated acid addition salt thereof.

2. 2,2-Dimethylpropanoyl-oxymethyl 7- [2-(2-aminothiaz-ol-4-yl)-2-(Z)-hydroxyimino-acetamido]-3-(2-methoxy-ethoxy-methyl)-3-cephem-4-carboxylate.

3. 2,2-Dimethylbutanoyl-oxymethyl 7-[2-(2-aminothiazol-4-yl)-2-(Z)-hydroxyimino-acetamido]-3-(2-methoxy-ethoxy-methyl)-3-cephem-4-carboxylate.

4. α-(2,2-Dimethylpropanoyl-oxy)-ethyl 7-[2-(2-aminothiazol-4-yl)-2-(Z)-hydroxyimino-acetamido]-3-(2-methoxyethoxy-methyl)-3-cephem-4-carboxylate.

5. α-(2,2-Dimethylbutanoyl-oxy)-ethyl 7-[2-(2-aminothiazol-4-yl)-2-(Z)-hydroxyimino-acetamido]-3-(2-methoxyethoxy-methyl)-3-cephem-4-carboxylate.

6. A process for the preparation of a cephemcarboxylic acid ester of the formula I 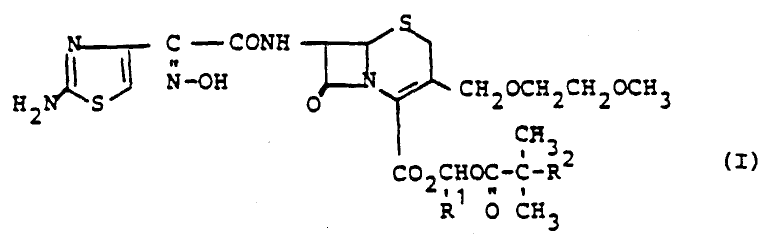 or a physiologically tolerated acid addition salt thereof, in which
R¹ = hydrogen or methyl,
R² = methyl or ethyl
and in which the HO group is in the syn-position, which comprises
a) reacting a compound of the formula II 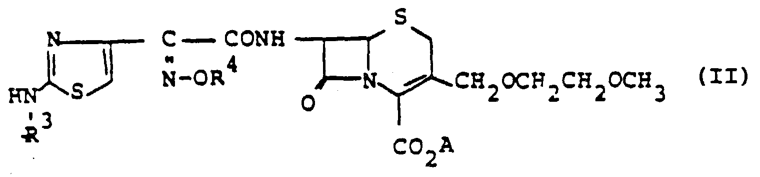 in which R³ represents an amino-protective group, R⁴ represents a group which can easily be split off and A represents a cation,
with a compound of the formula III 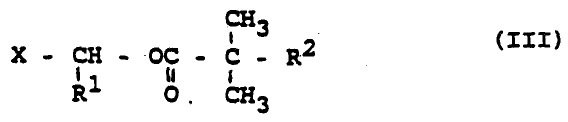 in which R¹ and R² have the above meaning and X represents a leaving group, to give the ester of the formula IV 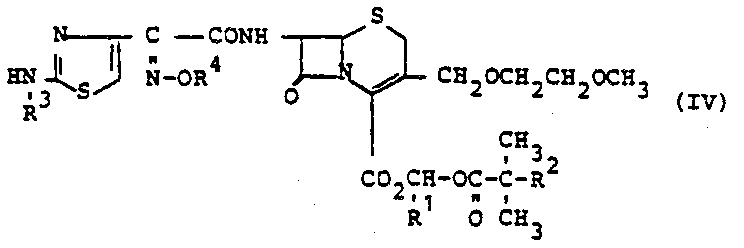 and removing the groups R³ and R⁴ in a manner which is known per se,
or
b) reacting a compound of the formula V 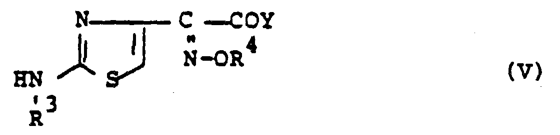 in which R³ and R⁴ have the above meaning and Y represents an activating group, with a compound of the formula VI 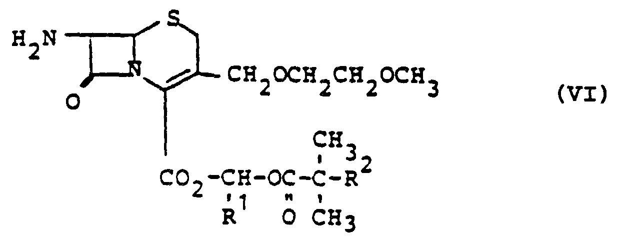 in which R¹ and R² have the above meaning, or with a salt of this compound, to give a compound of the formula IV, and splitting off the groups R³ and R⁴ in a manner which is known per se, or
c) reacting a compound of the formula VII 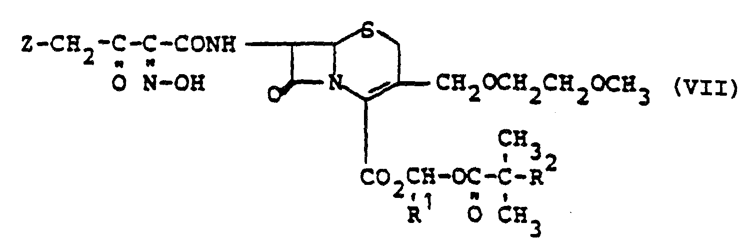 in which Z represents halogen and R¹ and R² have the above meaning, with thiourea to give a compound of the formula I,
and - if desired - converting the resulting compound into a physiologically tolerated acid addition salt.

7. A pharmaceutical formulation which is active against bacterial infections, containing a cephemcarboxylic acid ester of the formula I.

8. A process for the preparation of a pharmaceutical formulation which is active against bacterial infections, which comprises bringing a cephemcarboxylic acid ester of the formula I into a pharmaceutically suitable administration form using pharmaceutically customary excipients or diluents.

9. The use of a cephemcarboxylic acid ester of the formula I for preparing a pharmaceutical for combating bacterial infections.

## Claims (Claims for the following Contracting State(s): ES, GR)

1. A process for the preparation of a cephemcarboxylic acid ester of the formula I 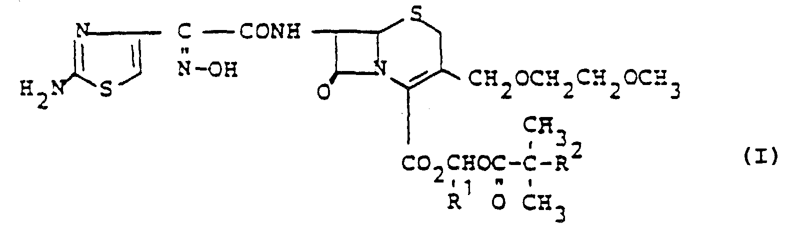 or a physiologically tolerated acid addition salt thereof, in which
R¹ = hydrogen or methyl,
R² = methyl or ethyl
and in which the HO group is in the syn-position, which comprises
a) reacting a compound of the formula II 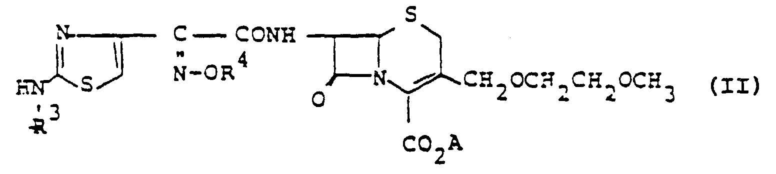 in which R³ represents an amino-protective group, R⁴ represents a group which can easily be split off and A represents a cation,
with a compound of the formula III 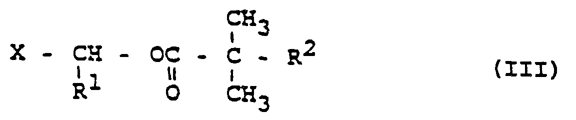 in which R¹ and R² have the above meaning and X represents a leaving group, to give the ester of the formula IV 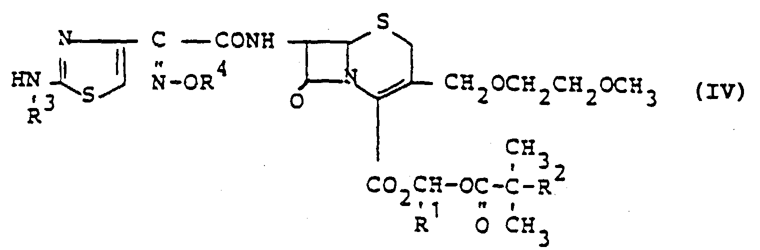 and removing the groups R³ and R⁴ in a manner which is known per se,
or
b) reacting a compound of the formula V 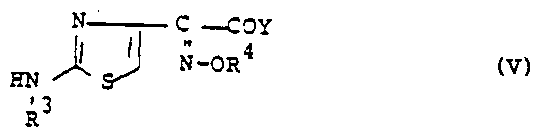 in which R³ and R⁴ have the above meaning and Y represents an activating group, with a compound of the formula VI 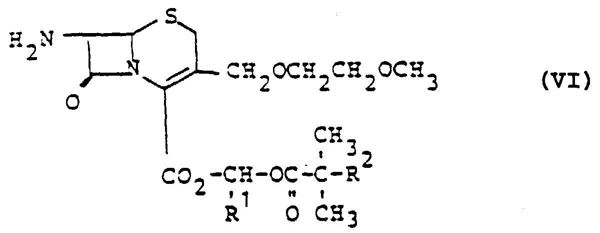 in which R¹ and R² have the above meaning, or with a salt of this compound, to give a compound of the formula IV, and splitting off the groups R³ and R⁴ in a manner which is known per se, or
c) reacting a compound of the formula VII 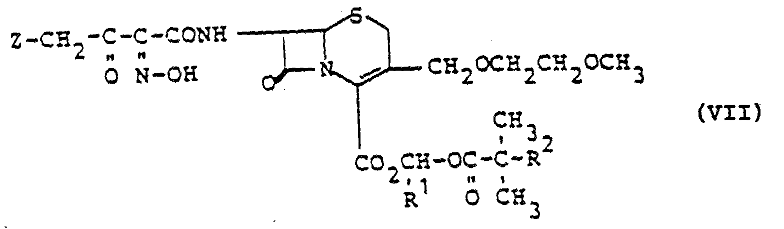 in which Z represents halogen and R¹ and R² have the above meaning, with thiourea to give a compound of the formula I,
and - if desired - converting the resulting compound into a physiologically tolerated acid addition salt.

2. A pharmaceutical formulation which is active against bacterial infections, containing a cephemcarboxylic acid ester of the formula I.

3. A process for the preparation of a pharmaceutical formulation which is active against bacterial infections, which comprises bringing a cephemcarboxylic acid ester of the formula I into a pharmaceutically suitable administration form using pharmaceutically customary excipients or diluents.

4. The use of a cephemcarboxylic acid ester of the formula I for preparing a pharmaceutical for combating bacterial infections.

## Revendications (Revendications pour l'(les) Etat(s) contractant(s) suivant(s): AT, BE, CH, DE, DK, FR, GB, IT, LI, LU, NL, SE)

1. Esters de l'acide céphème-carboxylique de Formule générale I 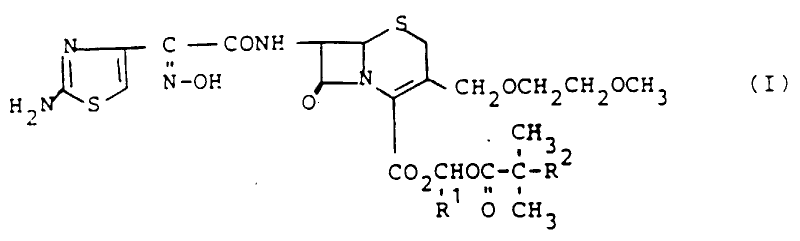 dans laquelle
R¹ représente un atome d'hydrogène ou le groupe méthyle,
R² représente un groupe méthyle ou éthyle,
et dans laquelle le groupe OH est en position syn, et leurs sels d'addition avec un acide physiologiquement acceptable.

2. Ester 2,2-diméthyl-propanoyl-oxy méthylique de l'acide 7-[2-(2-aminothiazol-4-yl)-2-(Z)-hydroxyimino-acétamido]-3-(2-méthoxyéthoxy-méthyl)-3-céphèm-4-carboxylique.

3. Ester 2,2-diméthyl-butanoyl-oxyméthylique de l'acide 7-[2-[2-aminothiazol-4-yl)-2-(Z)-hydroxyimino-acétamido]-3-(2-méthoxyéthoxy-méthyl)-3-céphèm-4-carboxylique.

4. Ester α-(2,2-diméthyl-propanoyl-oxy)éthylique de l'acide 7-[2-(2-aminothiazol-4-yl)-2-(Z)-hydroxyimino-acétamido]-3-(2-méthoxyéthoxy-méthyl)-3-céphém-4-carboxylique

5. Ester α-(2,2-diméthyl-butanoyl-oxy)éthylique de l'acide 7-[2-(2-aminothiazol-4-yl)-2-(Z)-hydroxyimino-acétamido]-3-(2-méthoxyéthoxy-méthyl)-3-céphèm-4-carboxylique

6. Procédé de fabrication d'esters de l'acide céphème-carboxylique de Formule générale I 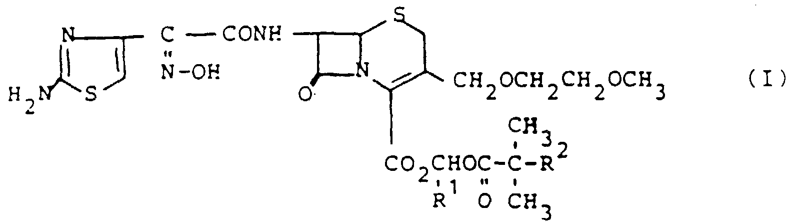 et de leurs sels d'addition avec un acide physiologiquement acceptable
dans laquelle
R¹ représente un antome d'hydrogène ou le groupe méthyle,
R² représente un groupe méthyle ou éthyle,
et dans laquelle le groupe OH est en position syn, caractérisé en ce que,
a) l'on fait réagir un composé de Formule II 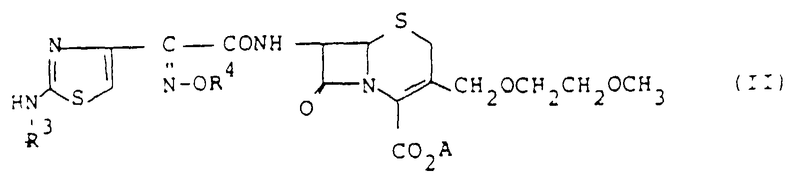 dans laquelle R¹ représente un groupe protecteur d'amine, R⁴ un groupe qui peut être éliminé facilement et A un cation,
avec un compose de Formule générale III 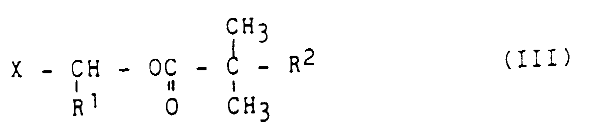 dans laquelle R¹ et R² possèdent la signification ci-dessus et X représente un groupe partant, pour donner l'ester de Formule générale IV 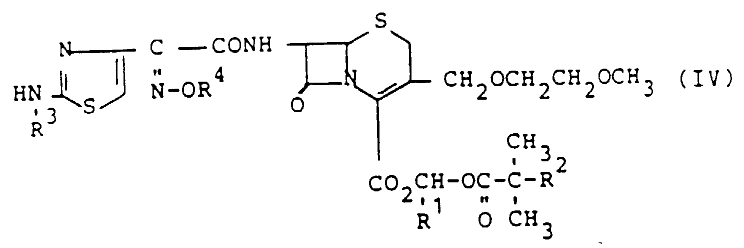 et l'on élimine les groupes R³ et R⁴ d'une manière connue en soi
ou
b) l'on fait réagir un composé de Formule générale V 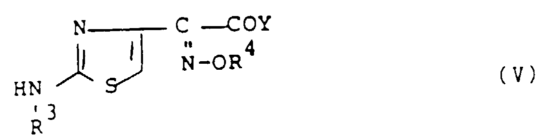 dans laquelle R³ et R⁴ possèdent la même signification que précédemment et Y représente un groupe d'activation, avec un composé de Formule générale VI 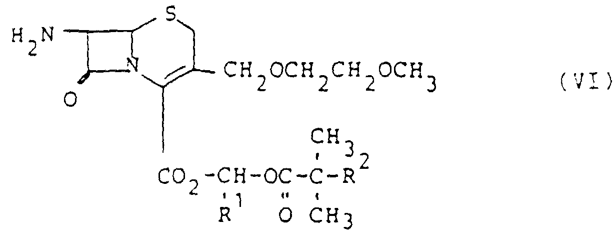 dans laquelle R¹ et R² possèdent la signification ci-dessus ou avec un sel de ce composé, pour donner un composé de Formule générale IV et l'on élimine les groupes R³ et R⁴ d'une manière connue en soi
ou
c) l'on fait réagir un composé de Formule générale VII 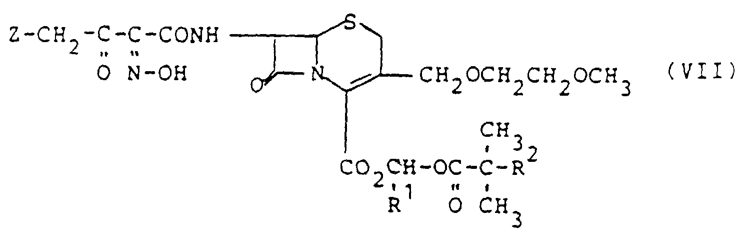 dans laquelle Z représente un atome d'halogène et R¹ et R² possèdent la même signification que précéàemment, avec un composé thiourée pour donner des composés de Formule générale I,
et - si on le souhaite - l'on transforme les composés obtenus en un sel d'addition avec un acide physiologiquement acceptable

7. Préparations pharmaceutiques actives contre les infections bactériennes caractérisées en ce qu'elles contiennent un ester d'acide céphème-carboxylique de Formule générale I.

8. Procédé de fabrication de préparations pharmaceutiques actives contre les infections bactériennes, caractérisé en ce que, l'on mélange un ester d'acide céphème-carboxylique de Formule générale I avec des véhicules pharmaceutiques ou des diluants courants dans une forme d'administration pharmaceutiquement appropriée.

9. Utilisation d'esters de l'acide céphème-carboxylique de Formule générale I pour la fabrication d'un médicament pour combattre les infections bactériennes.

## Revendications (Revendications pour l'(les) Etat(s) contractant(s) suivant(s): ES, GR)

1. Procédé de fabrication d'esters de l'acide céphème-carboxylique de Formule générale I 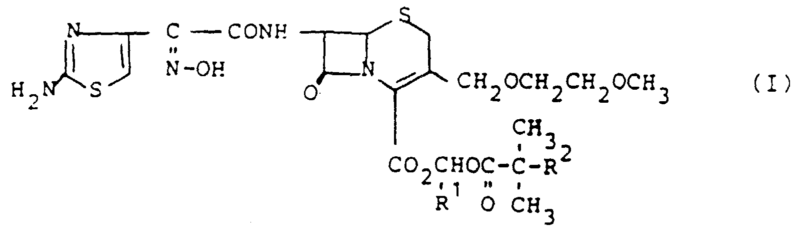 et de leurs sels d'addition avec un acide physiologiquement acceptable dans laquelle
R¹ représente un atome d'hydrogène ou le groupe méthyle,
R² représente un groupeméthyle ou éthyle,
et dans laquelle le groupe OH est en position syn, dans lequel,
a) on fait réagir un composé de Formule II 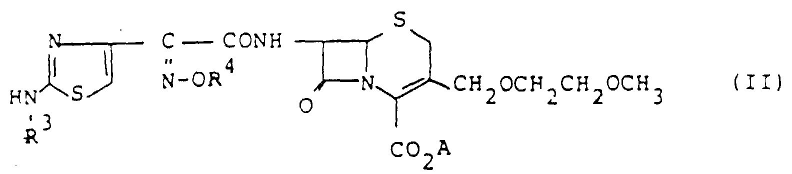 dans laquelle R³ représente un groupe protecteur d'amine, R⁴ un groupe qui peut être éliminé facilement et A un cation,
avec un composé de Formule générale III 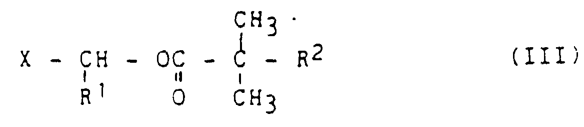 dans laquelle R¹ et R² possèdent la signification ci-dessus et X représente un groupe partant, pour donner l'ester de Formule générale IV 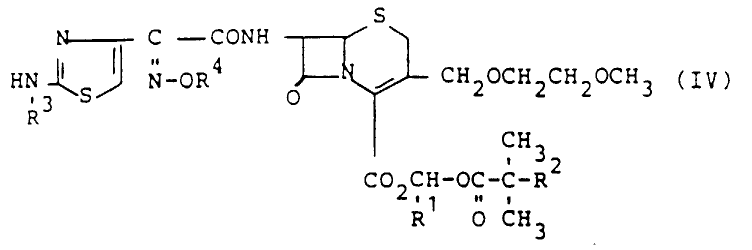 et l'on élimine les groupes R³ et R⁴ d'une manière connue en soi
ou
b) on fait réagir un composé de Formule générale V 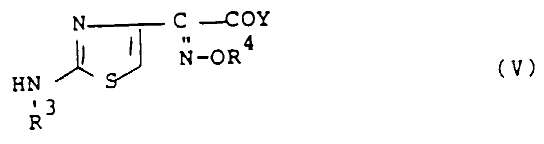 dans laquelle R³ et R⁴ possèdent la même signification que précédemment et Y représente un groupe d'activation, avec un composé de Formule générale VI 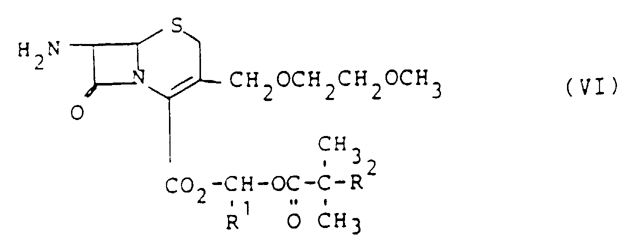 dans laquelle R¹ et R² possèdent la signification ci-dessus ou avec un sel de ce composé, pour donner un composé de Formule générale IV et l'on élimine les groupes R³ et R⁴ d'une manière connue en soi
ou
c) on fait réagir un composé de Formule générale VII 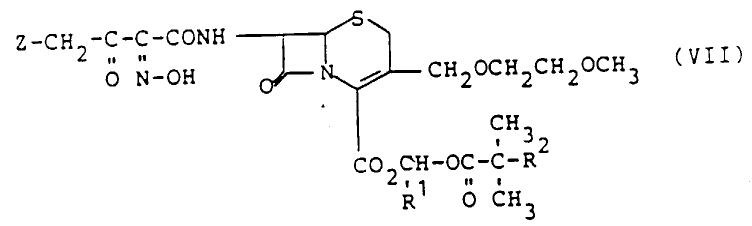 dans laquelle Z représente un atome d'halogène et R¹ et R² possèdent la même signification que précédemment, avec un composé thiourée pour donner des composés de Formule générale I,
et - si on le souhaite - l'on transforme les composés obtenus en un sel d'addition avec un acide physiologiquement acceptable

2. Préparations pharmaceutiques actives contre les infections bactériennes caractérisées en ce qu'elles contiennent un ester d'acide céphème-carboxylique de Formule générale I.

3. Procédé de fabrication de préparations pharmaceutiques actives contre les infections bactériennes, caractérisé en ce que, l'on mélange un ester d'acide céphème-carboxylique de Formule générale I avec des véhicules pharmaceutiques ou des diluants courants dans une forme d'administration pharmaceutiquement appropriée.

4. Utilisation d'esters de l'acide céphème-carboxylique de Formule générale I pour la fabrication d'un médicament pour combattre les infections bactériennes
